# EUROPEAN PATENT SPECIFICATION

(11) **EP 3 904 333 B1**
(45) Date of publication and mention of the grant of the patent: **04.12.2024**
(21) Application number: 20911028.7
(22) Date of filing: 30.06.2020
(51) Int. Cl.: C07C 227/18, C07C 229/50, C07C 227/32

(54) **METHOD FOR PREPARING (2S,3S)-3-AMINO-BICYCLO[2.2.2]OCTANE-2-CARBOXYLATE**
VERFAHREN ZUR HERSTELLUNG VON (2S3S)-3-AMINO-BICYCLO[ 2.2.2]OCTAN-2-CARBOXYLAT
PROCÉDÉ DE PRÉPARATION DE (2S,3S)-3-AMINO-BICYCLO[2.2.2]OCTANE-2-CARBOXYLATE

(30) Priority: 30.12.2019 CN 201911403717
(43) Date of publication of application: 03.11.2021
(73) Proprietor: Guangdong Raffles Pharmatech Co., Ltd, Huizhou, Guangdong 516081 (CN)
(72) Inventor: ZHOU, Zhangtao, Guangdong 516081 (CN); HUANG, Zhining, Guangdong 516081 (CN); YE, Weiping, Guangdong 516081 (CN); PHILLIS, Andrew Toroa, Guangdong 516081 (CN)
(74) Representative: Gille Hrabal Partnerschaftsgesellschaft mbB Patentanwälte
(86) International application number: PCT/CN2020/099360
(87) International publication number: WO 2021/135127

(56) References cited:
- WO-A1-2015/073481
- CN-A- 105 849 405
- CN-A- 109 678 738
- DAQIANG X ET AL: "A practical synthesis of enantiopure ethyl cis-2-amino-1-cyclohexanecarboxylate via asymmetric reductive amination methodology", TETRAHEDRON ASYMMETRY, PERGAMON PRESS LTD, OXFORD, GB, vol. 8, no. 9, 8 May 1997 (1997-05-08), pages 1445 - 1451, XP004064107, ISSN: 0957-4166, DOI: 10.1016/S0957-4166(97)00160-2
- DAQIANG, X. PRASAD, K. REPIC, O. BLACKLOCK, T.J.: "A practical synthesis of enantiopure ethyl cis-2-amino-1-cyclohexanecarboxylate via asymmetric reductive amination methodology", TETRAHEDRON ASYMMETRY, PERGAMON PRESS LTD, OXFORD, GB, vol. 8, no. 9, 8 May 1997 (1997-05-08), OXFORD, GB, pages 1445 - 1451, XP004064107, ISSN: 0957-4166, DOI: 10.1016/S0957-4166(97)00160-2
- KISS LORÁND, KARDOS MÁRTON, FORRÓ ENIKÖ, FÜLÖP FERENC: "Stereocontrolled One-Step Synthesis of Difunctionalised Cispentacin Derivatives through Ring-Opening Metathesis of Norbornene β-Amino Acids : Synthesis of Difunctionalised Cispentacin Derivatives", EUROPEAN JOURNAL OF ORGANIC CHEMISTRY, WILEY-VCH, DE, vol. 2015, no. 6, 1 February 2015 (2015-02-01), DE, pages 1283 - 1289, XP055826118, ISSN: 1434-193X, DOI: 10.1002/ejoc.201403493

## Description

The present application claims the priority of the prior application No. 201911403717.X submitted to China National Intellectual Property Administration on December 30, 2019, which is entitled "Method for preparing (2S,3S)-3-amino-bicyclo[2.2.2]octane-2-carboxylate".

### Field of the Invention

The present disclosure belongs to the field of organic chemical synthesis of pharmaceutical intermediates, and specifically relates to a new method for the synthesis and industrialization of (2S,3S)-3-amino-bicyclo[2.2.2]octane-2-carboxylate using an in-house designed and innovative intermediate.

### Background of the Invention

(2S,3S)-3-amino-bicyclo[2.2.2]octane-2-carboxylate belongs to a class of chiral small molecules that are difficult to synthesize. This chiral fragment is widely used in the manufacture of an influenza virus RNA polymerase inhibitors, the most representative of which is Pimodivir developed by Vertex, which has entered the clinical phase III study. The novel target of this class of drugs is a milestone in addressing influenza virus drug resistance.

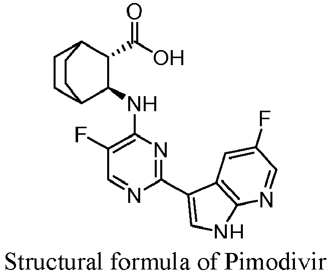

Three main routes have been reported for the synthesis of this structural fragment of (2S,3S)-3-amino-bicyclo[2.2.2]octane-2-carboxylate as follows.

Route I is shown as the following chart "Route I reported in a patent". This route starts with cyclohexadiene, undergoes Diels-Alder reaction with maleic anhydride, further selective alcoholysis in the presence of quinidine to obtain cis-carboxylic acid esters, flips the ester group conformation under strong base conditions, and finally performs Curtius rearrangement with diphenyl azide phosphate, and finally obtain the target product by removal of benzoxy carbonyl. Although the starting material is relatively cheap, the main shortcomings of this route include:
(1) requires the use of expensive chiral organic base quinidine for desymmetrizing alcoholysis, resulting in high costs;
(2) requires the use of explosive azide compounds, which is a safety hazard and not conducive to scaling up production;
(3) the total yield is less than 20%.

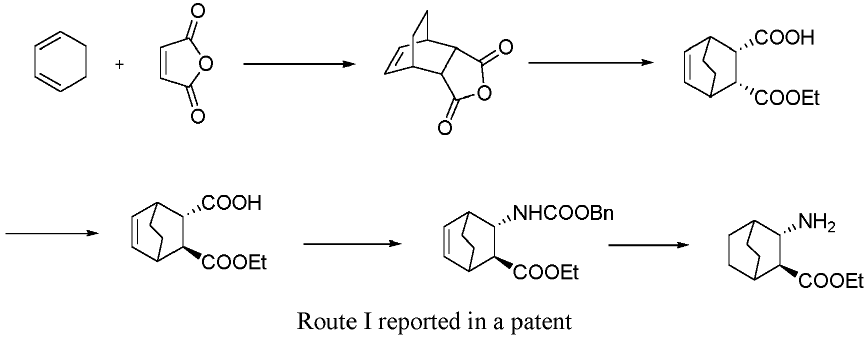

Route II is shown as the following chart "Route II reported in a patent". This route takes cyclohexadiene as the starting material, undergoes the Diels-Alder reaction with ethyl propargylate, and after further hydrogenation for selective reduction of the double bond, undergoes Michael addition reaction with chiral amine anion at low temperature, and finally removes the two protecting groups to obtain the target compound.

This route also has many shortcomings. Firstly, the starting materials are expensive and costly; secondly, special metal catalysts are used in the selective reduction of double bonds and harsh conditions such as ultra-low temperatures are used in the subsequent addition reactions, which are not conducive to scale-up production; the overall production cost is also high.

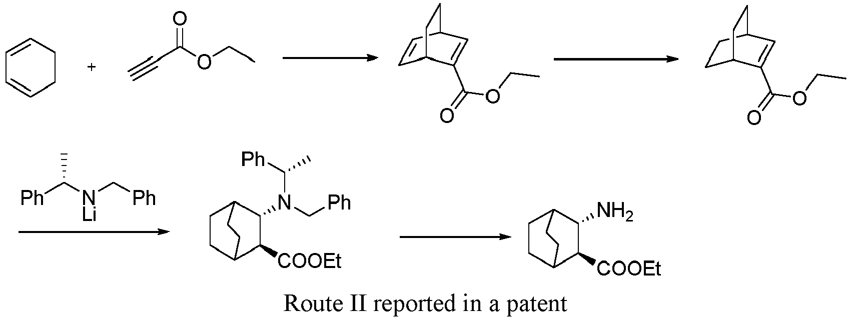

Route III is shown as the following chart "Route III reported in a patent". This route starts with ethyl glyoxylate, which undergoes the Henry reaction with nitromethane under alkaline conditions to obtain ethyl nitroacrylate by elimination reaction, which is further hydrogenated with cyclohexadiene by the Diels-Alder reaction catalyzed by chiral auxiliaries to obtain the target product.

Although this route is short, the raw materials are expensive and chemically unstable, while the use of nitromethane and nitro-containing intermediates poses a greater safety risk to the production.

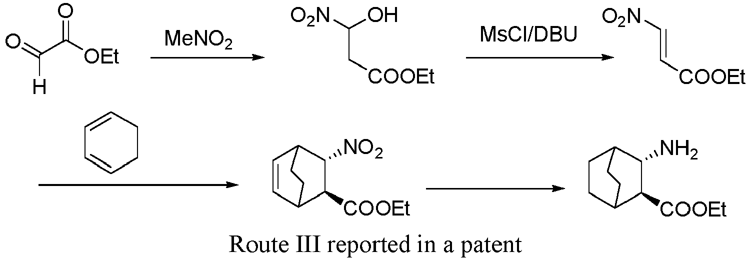

In summary, the reported synthetic routes for (2S,3S)-3-amino-bicyclo[2.2.2]octane-2-carboxylate are characterized by high production risks and high costs, which make it difficult to meet the demand for this class of pharmaceutical intermediates in the pharmaceutical industry. WO2015/073481 discloses the preparation of a compound of formula I via reaction of a compound of formula (C) with diphenylphosphoryl azide and benzyl alcohol to form a compound of formula (D) which is then hydrogenated to obtain the expected product.

### Summary of the Invention

To address the shortcomings of the prior art and to solve the problems of high preparation cost, low material safety and difficulty in production scale-up of (2S,3S)-3-amino-bicyclo[2.2.2]octane-2-carboxylate, the present invention provides a method for the preparation of (2S,3 S)-3-amino-bicyclo[2.2.2]octane-2-carboxylate through two independently designed and innovative intermediates. The method utilizes a chiral reductive amination strategy and achieves excellent results.

The present disclosure provides a method for preparing (2S,3S)-3-amino-bicyclo[2.2.2]octane-2-carboxylate, the method comprises: using 3-carbonyl-bicyclo[2.2.2]octane-2-carboxylate as raw material, carring out reductive amination, flip of ester group conformation and removal of protecting group to obtain the (2S,3S)-3-amino-bicyclo[2.2.2]octane-2-carboxylate, and reaction process is shown below, 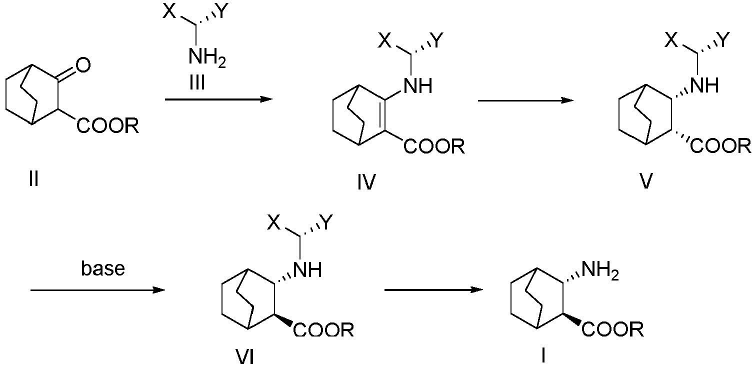 wherein the method comprises:
S1, reacting 3-carbonyl-bicyclo[2.2.2]octane-2-carboxylate with chiral amines in presence of acid to give 3-amido-bicyclo[2.2.2]octene-2-carboxylate;
S2, carrying out reduction with a reducing agent or metal-catalyzed hydrogenation of the 3-amido-bicyclo[2.2.2]octene-2-carboxylate to give (2R,3S)-3-amido-bicyclo[2.2.2]octane-2-carboxylate;
S3, under strong base conditions, carrying out ester configuration flip of the (2R,3 S)-3-amido-bicyclo[2.2.2]octane-2-carboxylate to give (2S,3S)-3-amido-bicyclo[2.2.2]octane-2-carboxylate;
S4, carrying out hydrogenation of the (2S,3S)-3-amido-bicyclo[2.2.2]octane-2-carboxylate to remove the protecting group to give the (2S,3S)-3-amino-bicyclo[2.2.2]octane-2-carboxylate. R is methyl, ethyl, propyl, butyl, phenyl or benzyl; the X is methyl, ethyl, phenyl or 1-naphthyl; the Y is methyl, ethyl, phenyl or 1-naphthyl; and the X and the Y are not identical, the X group is larger than the Y group. By "the X group is larger than the Y group", it means that mass of the X group is greater than mass of the Y group, or relative molecular weight of the X group is greater than relative molecular weight of the Y group. In the above selection range of X and Y groups, the relative molecular weight of the X group is larger than that of the Y group, which makes the steric effect of the X group larger than that of the Y group, and the chiral amine (compound III) is of S-configuration, which is ultimately favorable to obtain a chiral S-configuration product.

According to an embodiment of the present invention, the X is preferably phenyl, the Y is preferably methyl and the R is preferably ethyl considering the availability of the materials.

According to an embodiment of the present invention, in the S 1, the metal catalyst for hydrogenation reduction comprises one of platinum carbon, platinum dioxide and ruthenium metal catalyst; the reduction reagent comprises one of sodium borohydride, sodium triacetoxy borohydride, sodium cyanoborohydride and sodium trifluoroacetoxy borohydride.

According to an embodiment of the present invention, in the S2, the metal-catalyzed hydrogenation is carried out using a metal catalyst, the metal catalyst comprising at least one selected from the group consisting of platinum carbon, platinum dioxide and ruthenium metal catalyst; the reducing agent comprising at least one selected from the group consisting of sodium borohydride, sodium triacetoxy borohydride, sodium cyanoborohydride and sodium trifluoroacetoxy borohydride; preferably, to further improve the selectivity of the reaction, the metal catalyst is platinum dioxide; the reducing agent is sodium triacetoxy borohydride.

According to an embodiment of the present invention, in the S3, the strong base comprises at least one selected from the group consisting of sodium tert-butoxide, sodium tert-amylate, lithium diisopropylamide, lithium bis(trimethylsilyl)amide, sodium bis(trimethylsilyl)amide and potassium bis(trimethylsilyl)amide; preferably, considering price and availability of materials, the strong base is sodium tert-butoxide.

According to an embodiment of the present invention, in the S 1, the acid is organic acid or inorganic acid; preferably, the acid is strong organic acid; further preferably, the acid comprises p-toluenesulfonic acid or trifluoroacetic acid.

The present disclosure provides a compound shown in formula V below, wherein, the R is methyl, ethyl, propyl, butyl, phenyl or benzyl, preferably ethyl; the X is methyl, ethyl, phenyl or 1-naphthyl, and the Y is methyl, ethyl, phenyl or 1-naphthyl, and the X and the Y are not identical and the X is larger than the Y, preferably, the X is phenyl, the Y is methyl,

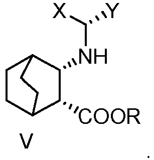

The present disclosure provides use of the compound V for preparation of (2S,3S)-3-amino-bicyclo[2.2.2]octane-2-carboxylate.

The present disclosure provides a compound shown in formula VI below, wherein, the R is methyl, ethyl, propyl, butyl, phenyl or benzyl, preferably ethyl; the X is methyl, ethyl, phenyl or 1-naphthyl, and the Y is methyl, ethyl, phenyl or 1-naphthyl, and the X and the Y are not identical and the X is larger than the Y, preferably, the X is phenyl, the Y is methyl,

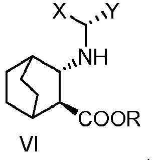

The present disclosure provides use of the compound VI for preparation of (2S,3S)-3-amino-bicyclo[2.2.2]octane-2-carboxylate.

Compared with the prior art, the present invention has the following advantages.
1. The synthetic route of the present invention is a novel route for the preparing of (2S,3S)-3-amino-bicyclo[2.2.2]octane-2-carboxylate. Two novel intermediates, namely, compound V and compound VI are obtained through the synthetic route of the present invention. An overall yield of more than 65% is achieved by the synthetic route of the present invention. The synthetic route of the present invention is also characterized by the short route with relatively mild reaction conditions.
2. High chiral purity intermediates can be obtained by the synthetic route provided by the present invention. The chiral purity of the target products can be increased to more than 99.5% after simple crystallization and purification.
3. Easily available raw materials are employed in the processes. The synthetic route of the present invention is low cost, requires no special operating process, employs simple equipments, and is suitable for large-scale industrial production.

### Detailed Description of the Invention

The following is a detailed description of preferred embodiments of the present invention to make the advantages and features of the present invention more easily understood by those skilled in the art, so that the scope of protection of the present invention can be more clearly defined.

### Example 1

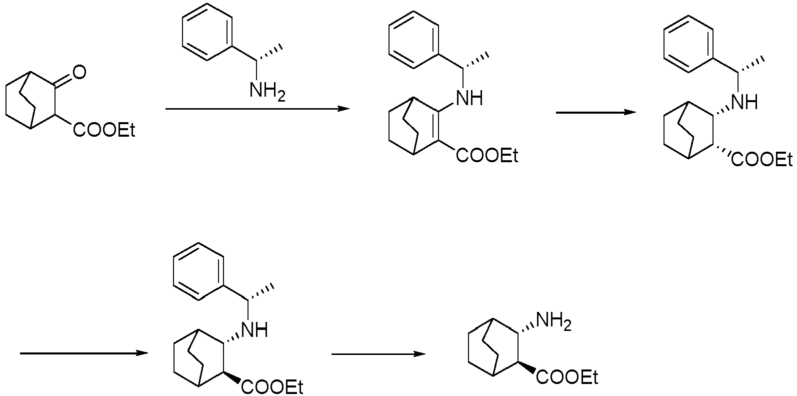

### 1. Synthesis of ethyl (S) -3-(1-phenylethylamino)-bicyclo[2.2.2]octene-2-carboxylate

To the reactor was added 1000 L toluene, 100.0 kg ethyl 3-carbonyl-bicyclo[2.2.2]octane-2-carboxylate, 12 kg p-toluenesulfonic acid, 80.0 kg S-1-phenylethylamine, and the reaction was refluxed under nitrogen protection for 12 h to obtain the enamine intermediate ethyl (S) -3-(1-phenylethylamino)-bicyclo[2.2.2]octene-2-carboxylate, which was used in the next reaction step.

### 2. Synthesis of ethyl (2R,3S)-3-((S)-1-phenylethylamino)-bicyclo[2.2.2]octane-2-carboxylate

The above enamine intermediate (S)ethyl-3-(1-phenylethylamino)-bicyclo[2.2.2]octene-2-carboxylate solution was desolvated, then 1500 L of tetrahydrofuran and 500 L of acetic acid was added, then 106.2 kg of sodium triacetoxyborohydride was added after cooling. Bring to room temperature and reacted for 3 h. 3N sodium hydroxide solution was added dropwise to adjust to alkaline, extracting with ethyl acetate (800 Lx 2), the combined organic phases were washed with saturated salt water and concentrated to give 115 g of ethyl (2R,3S)-3-((S)-1-phenylethylamino)-bicyclo[2.2.2]octane-2-carboxylate (corresponding to compound IV in the synthetic route of the present invention) as a pale yellow oil, in yield 85%. ¹HNMR (400 MHz, CDCl₃) 67.22-7.32 (m, 5H), δ4.18 (q, 2H), δ3.65 (q, 1H), δ2.81-2.89 (m, 2H), δ1.83 (m, 2H) , δ1.27-1.56 (m, 11H) , δ1.25 (t, 3H) ; ESI-MS: m/z 302.34 [M+1]

### 3. Synthesis of ethyl (2S,3S)-3-((S)-1-phenylethylamino)-bicyclo[2.2.2]octane-2-carboxylate

To the reactor was added 500 L tetrahydrofuran, 500 L tert-butanol, 64 kg sodium tert-butoxide, and cooled to 0-10 °C under nitrogen protection. Add tetrahydrofuran solution of ethyl (2R,3S)-3-((S)-1-phenylethylamino)-bicyclo[2.2.2]octane-2-carboxylate (100 kg dissolved in 100 L tetrahydrofuran) dropwise. After dropwise addition, the reaction was held for 2 h. The reaction solution was transferred to 500 L saturated ammonium chloride solution for quenching. After extraction with ethyl acetate (800 L x 2), the combined organic phases were washed with saturated brine and concentrated to give 90.0 kg of ethyl (2S,3S)-3-((S)-1-phenylethylamino)-bicyclo[2.2.2]octane-2-carboxylate (corresponding to compound V in the synthetic route of the present invention) as pale yellow oily form with 90.0% yield and diastereomeric purity 97.4%. ¹HNMR (400 MHz, CDCl₃) δ7.21-7.31 (m, 5H), δ4.13 (q, 2H), δ3.79 (q, 1H), δ3.12 (d, 1H) , δ2.22 (d, 1H) , δ1.93 (d, 1H) ,δ1.42-1.76 (m, 8H), δ1.23-1.34 (m, 8H) ; ESI-MS: m/z 302.34 [M+1]

### 4. Synthesis of ethyl (2S,3S)-3-amino-bicyclo[2.2.2]octane-2-carboxylate

500 L of ethanol, 8.00 kg of ethyl (2S,3S)-3-((S)-1-phenylethylamino)-bicyclo[2.2.2]octane-2-carboxylate, and 8 kg of 10% palladium carbon were added to a 1000 L stainless steel autoclave.The reactor was evacuated and full filled with nitrogen, then exchanged to hydrogen and pressurized to to 0.6 MPa.The reaction mixture was heated to 50°C and kept for 12 hours. The palladium carbon was removed by filtration and the filtrate was concentrated to give 50 kg of ethyl (2S,3S)-3-amino-bicyclo[2.2.2]octane-2-carboxylate (corresponding to compound I in the synthetic route of the present invention) as a pale yellow oil in 97.0% yield and 97.5% chiral purity.
¹HNMR (400 MHz, CDCl₃) δ4.18 (q, 2H), δ3.37-3.38 (m, 1H), δ2.13-2.17 (m, 3H), δ1.98-2.00 (m, 1H) , δ1.78-1.83 (m, 1H) , δ1.36-1.67 (m, 9H) ,δ1.27 (t, 3H) ; ESI-MS: m/z 198.26 [M+1]

### Example 2

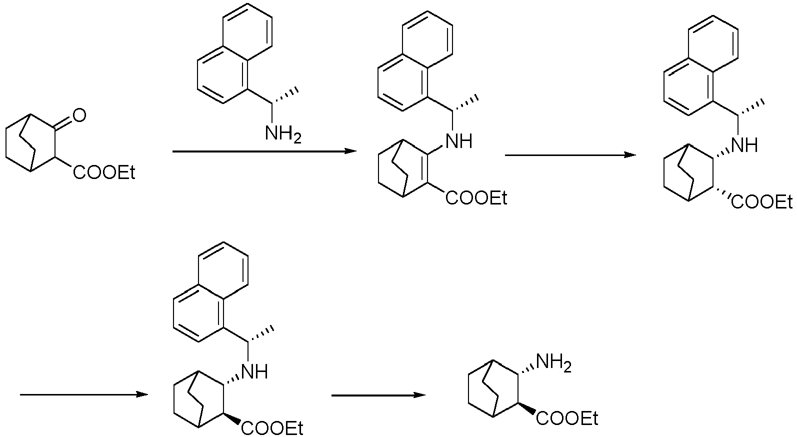

### 1. Synthesis of ethyl (S)-3-(1-naphthylethylamino)-bicyclo[2.2.2]octene-2-carboxylate

1000 L of toluene, 100.0 kg of ethyl 3-carbonyl-bicyclo[2.2.2]octane-2-carboxylate, 10.0 kg of trifluoroacetic acid, 113 kg of S-1-naphthyl ethylamine were added to the reactor and reacted under nitrogen protection at reflux for 12 hours. Cooled to room temperature, washed with 300 L saturated sodium bicarbonate solution, the organic layer was concentrated to 200 L, 500 L n-heptane was added and stirred for 3h at room temperature, filtered, washed with a small amount of n-heptane and dried under vacuum to give 133.6 kg ethyl (S)-3-(1-naphthylethylamino)-bicyclo[2.2.2]octene-2-carboxylate as a white solid in 75.3% yield.

### 2. Synthesis of ethyl (2R,3S)-3-((S)-1-naphthylethylamino)-bicyclo[2.2.2] octane-2-carboxylate

300 L of ethanol, 200 L of ethyl acetate, 100.0 kg of ethyl (S)-3-(1-naphthylethylamino)-bicyclo[2.2.2]octene-2-carboxylate, 10.0 kg of 5% platinum carbon to 1000 L were added to stainless steel autoclave, degassing with nitrogen and then ventilating with hydrogen to 1 MPa, then the mixture was raised to 35 °C and kept for 10 hours. Filtered to remove the platinum carbon and the filtrate was concentrated to obtain 100.0 kg ethyl (2R,3S)-3-((S)-1-naphthylethylamino)-bicyclo[2.2.2] octane-2-carboxylate as an off-white solid in 99.4% yield.

### 3. Synthesis of ethyl (2S,3S)-3-((S)-1-naphthylethylamino)-bicyclo[2.2.2]octane-2-carboxylate

500 L tetrahydrofuran, 500 L tert-butanol, 50.0 kg sodium tert-butoxide were added to the reactor, cooled down to 0-10°C under nitrogen protection, and added dropwise a tetrahydrofuran solution of ethyl (2R,3S)-3-((S)-1-naphthylethylamino)-bicyclo[2.2.2] octane-2-carboxylate (100.0 kg dissolved in 100 L tetrahydrofuran). After dropwise addition, the reaction was held for 2 h. The reaction solution was quenched by pouring into 500 L saturated ammonium chloride solution, then extracted with ethyl acetate (800 L x 2). The combined organic phases were washed with saturated brine and concentrated to give 92.0 kg of ethyl (2S,3S)-3-((S)-1-naphthylethylamino)-bicyclo[2.2.2]octane-2-carboxylate as a pale yellow solid in 92.0% yield and 98.0% diastereomeric purity.

### 4. Synthesis of ethyl (2S,3S)-3-amino-bicyclo[2.2.2]octane-2-carboxylate

To a 1000 L stainless steel hydrogenator was added 500 L of ethanol, 90.0 kg of ethyl (2S,3S)-3-((S)-1-naphthylethylamino)-bicyclo[2.2.2]octane-2-carboxylate, 9 kg of 10% palladium carbon. After degassing with nitrogen, then ventilating with hydrogen to 1 MPa and the reaction was carried out at 50 °C for 12 h. The palladium carbon was removed by filtration and the filtrate was concentrated to give 50.0 kg of ethyl (2S,3S)-3-amino-bicyclo[2.2.2]octane-2-carboxylate as a pale yellow oil in 99.0% yield and 98.1% chiral purity.

## Claims

1. A method for preparing a (2S,3S)-3-amino-bicyclo[2.2.2]octane-2-carboxylate, wherein, the method comprises: using a 3-carbonyl-bicyclo[2.2.2]octane-2-carboxylate of formular II as a raw material, carring out reductive amination, epimerization and removal of protecting group to obtain the (2S,3S)-3-amino-bicyclo[2.2.2]octane-2-carboxylate of formular I, and the reaction process is shown below, 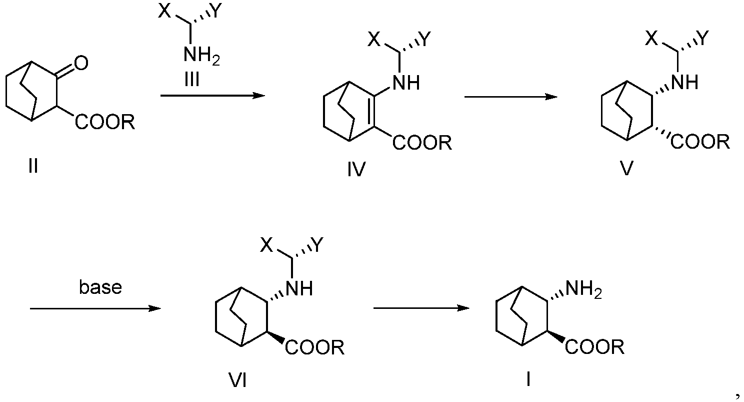 wherein,
the R is methyl, ethyl, propyl, butyl, phenyl or benzyl, the X is methyl, ethyl, phenyl or 1-naphthyl, the Y is methyl, ethyl, phenyl or 1-naphthyl, and the X and the Y are not identical, the X group is larger than the Y group;
the method comprises:
S1, reacting the 3-carbonyl-bicyclo[2.2.2]octane-2-carboxylate with the chiral amine of formula (III) in the presence of an acid to give the 3-amino-bicyclo[2.2.2]octene-2-carboxylate, the acid is a strong organic acid;
S2, carrying out reduction with a reducing agent or metal-catalyzed hydrogenation of the 3-amino-bicyclo[2.2.2]octene-2-carboxylate to give the (2R,3S)-3-amino-bicyclo[2.2.2]octane-2-carboxylate;
S3, under strong base conditions, carrying out the ester configuration flip of the (2R,3S)-3-amino-bicyclo[2.2.2]octane-2-carboxylate to give the (2S,3S)-3-amino-bicyclo[2.2.2]octane-2-carboxylate, the strong base comprises at least one selected from the group consisting of sodium tert-butoxide, sodium tert-amylate, lithium diisopropylamide, lithium bis(trimethylsilyl)amide, sodium bis(trimethylsilyl)amide and potassium bis(trimethylsilyl)amide; and
S4, carrying out hydrogenation of the (2S,3S)-3-amino-bicyclo[2.2.2]octane-2-carboxylate of formula VI to remove the protecting group to give the (2S,3S)-3-amino-bicyclo[2.2.2]octane-2-carboxylate of formula I.

2. The method according to claim 1, wherein, the R is ethyl.

3. The method according to claim 1, wherein, in the S2, the metal-catalyzed hydrogenation is carried out using a metal catalyst, the metal catalyst comprising at least one selected from the group consisting of platinum carbon, platinum dioxide and ruthenium metal catalyst; or the reduction is carried out using the reducing agent, the reducing agent comprising at least one selected from the group consisting of sodium borohydride, sodium triacetoxy borohydride, sodium cyanoborohydride and sodium trifluoroacetoxy borohydride.

4. The method according to claim 1, wherein, in the S3,the strong base is sodium tert-butoxide.

5. The method according to claim 1, wherein, in the S1, the acid comprises p-toluenesulfonic acid or trifluoroacetic acid.

6. A compound of formula V, wherein, the R is methyl, ethyl, propyl, butyl, phenyl or benzyl; the X is methyl, ethyl, phenyl or 1-naphthyl, and the Y is methyl, ethyl, phenyl or 1-naphthyl, and the X and the Y are not identical and the X is larger than the Y,

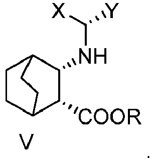

7. A method for preparation of (2S,3S)-3-amino-bicyclo[2.2.2]octane-2-carboxylate, wherein, the compound V according to claim 6 is used in the method.

8. A compound of formula VI, wherein, the R is methyl, ethyl, propyl, butyl, phenyl or benzyl; the X is methyl, ethyl, phenyl or 1-naphthyl, and the Y is methyl, ethyl, phenyl or 1-naphthyl, and the X and the Y are not identical and the X is larger than the Y,

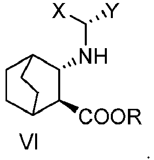

9. A method for preparation of (2S,3S)-3-amino-bicyclo[2.2.2]octane-2-carboxylate, wherein, the compound VI according to claim 8 is used in the method.

10. The method according to claim 1, wherein, the X is phenyl, the Y is methyl.

11. The method according to claim 3, wherein, the metal catalyst is platinum dioxide; the reducing agent is sodium triacetoxy borohydride.

12. (New) The compound of claim 6, wherein, the R is ethyl; the X is phenyl, the Y is methyl.

13. (New) The compound of claim 8, wherein, the R is ethyl; the X is phenyl, the Y is methyl.

## Patentansprüche

1. Verfahren zur Herstellung eines (2S,3S)-3-Amino-bicyclo[2.2.2]octan-2-carboxylats, wobei das Verfahren Folgendes umfasst: Verwenden eines 3-Carbonyl-bicyclo[2.2.2]octan-2-carboxylats der Formel II als Rohmaterial, Durchführen einer reduktiven Aminierung, Epimerisierung und Entfernung einer Schutzgruppe, um das (2S,3S)-3-Amino-bicyclo[2.2.2]octan-2-carboxylat der Formel I zu erhalten, und der Reaktionsprozess nachstehend gezeigt wird, 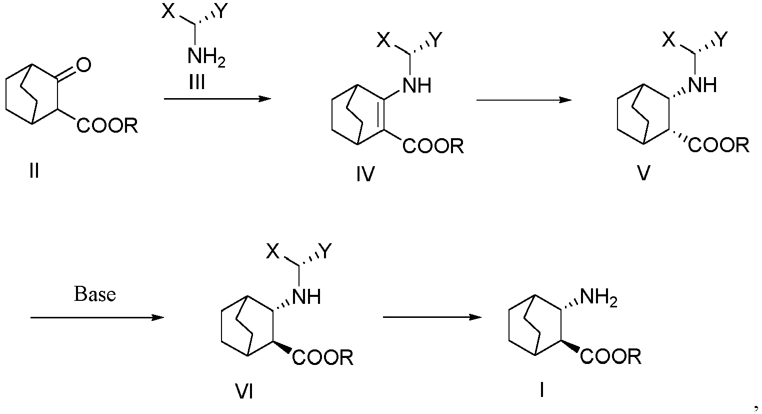 wobei
es sich bei R um Methyl, Ethyl, Propyl, Butyl, Phenyl oder Benzyl handelt, bei X um Methyl, Ethyl, Phenyl oder 1-Naphthyl handelt, bei Y um Methyl, Ethyl, Phenyl oder 1-Naphthyl handelt, und X und Y nicht identisch sind, die X-Gruppe größer ist als die Y-Gruppe;
wobei das Verfahren Folgendes umfasst:
S1, Umsetzen des 3-Carbonyl-bicyclo[2.2.2]octan-2-carboxylats mit dem chiralen Amin der Formel (III) in Gegenwart einer Säure, um das 3-Amino-bicyclo[2.2.2]octen-2-carboxylat zu erhalten, wobei es sich bei der Säure um eine starke organische Säure handelt;
S2, Durchführen einer Reduktion mit einem Reduktionsmittel oder metallkatalysierten Hydrierung des 3-Amino-bicyclo[2.2.2]octen-2-carboxylats, um das (2R,3S)-3-Amino-bicyclo[2.2.2]octan-2-carboxylat zu erhalten;
S3, Durchführen des Ester-Konfigurationswechsels des (2R,3S)-3-Amino-bicyclo[2.2.2]octan-2-carboxylats, unter stark basischen Bedingungen, um das (2S,3S)-3-Amino-bicyclo[2.2.2]octan-2-carboxylat zu erhalten, wobei die starke Base zumindest eines ausgewählt aus der Gruppe bestehend aus Natrium-tert-butoxid, Natrium-tert-amylat, Lithiumdiisopropylamid, Lithium-bis(trimethylsilyl)amid, Natrium-bis(trimethylsilyl)amid und Kalium-bis(trimethylsilyl)amid umfasst; und
S4, Durchführen einer Hydrierung des (2S,3S)-3-Amino-bicyclo[2.2.2]octan-2-carboxylats der Formel VI, um die Schutzgruppe zu entfernen, um das (2S,3S)-3-Amino-bicyclo[2.2.2]octan-2-carboxylat der Formel I zu erhalten.

2. Verfahren nach Anspruch 1, wobei es sich bei R um Ethyl handelt.

3. Verfahren nach Anspruch 1, wobei in S2 die metallkatalysierte Hydrierung unter Verwendung eines Metallkatalysators durchgeführt wird, wobei der Metallkatalysator zumindest eines ausgewählt aus der Gruppe bestehend aus Platinkohlenstoff-, Platindioxid- und Ruthenium-Metallkatalysator umfasst; oder die Reduktion unter Verwendung des Reduktionsmittels durchgeführt wird, wobei das Reduktionsmittel zumindest eines ausgewählt aus der Gruppe bestehend aus Natriumborhydrid, Natriumtriacetoxyborhydrid, Natriumcyanoborhydrid und Natriumtrifluoracetoxyborhydrid umfasst.

4. Verfahren nach Anspruch 1, wobei es sich bei der starken Base in S3 um Natrium-tert-butoxid handelt.

5. Verfahren nach Anspruch 1, wobei in S1 die Säure p-Toluolsulfonsäure oder Trifluoressigsäure umfasst.

6. Verbindung der Formel V, wobei es sich bei R um Methyl, Ethyl, Propyl, Butyl, Phenyl oder Benzyl handelt; bei X um Methyl, Ethyl, Phenyl oder 1-Naphthyl handelt und bei Y um Methyl, Ethyl, Phenyl oder 1-Naphthyl handelt, und das X und das Y nicht identisch sind und das X größer ist als das Y,

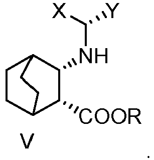

7. Verfahren zur Herstellung von (2S,3S)-3-Amino-bicyclo[2.2.2]octan-2-carboxylat, wobei die Verbindung V nach Anspruch 6 in dem Verfahren verwendet wird.

8. Verbindung der Formel VI, wobei es sich bei R um Methyl, Ethyl, Propyl, Butyl, Phenyl oder Benzyl handelt; bei X um Methyl, Ethyl, Phenyl oder 1-Naphthyl handelt und bei Y um Methyl, Ethyl, Phenyl oder 1-Naphthyl handelt, und das X und das Y nicht identisch sind und das X größer ist als das Y,

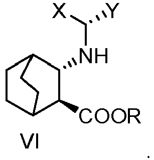

9. Verfahren zur Herstellung von (2S,3S)-3-Amino-bicyclo[2.2.2]octan-2-carboxylat, wobei die Verbindung VI nach Anspruch 8 in dem Verfahren verwendet wird.

10. Verfahren nach Anspruch 1, wobei es sich bei X um Phenyl handelt und bei Y um Methyl handelt.

11. Verfahren nach Anspruch 3, wobei es sich bei dem Metallkatalysator um Platindioxid handelt und bei dem Reduktionsmittel um Natriumtriacetoxyborhydrid handelt.

12. Verbindung nach Anspruch 6, wobei es sich bei R um Ethyl handelt, bei X um Phenyl handelt und bei Y um Methyl handelt.

13. Verbindung nach Anspruch 8, wobei es sich bei R um Ethyl handelt, bei X um Phenyl handelt und bei Y um Methyl handelt.

## Revendications

1. Procédé de préparation d'un (2S,3S)-3-amino-bicyclo[2.2.2]octane-2-carboxylate, dans lequel, le procédé comprend : l'utilisation d'un 3-carbonyl-bicyc lo[2.2.2]octane-2-carboxylate de formule II comme matière première, la réalisation d'une amination réductrice, d'une épimérisation et l'élimination du groupe protecteur pour obtenir le (2S,3S)-3-amino-bicyclo[2.2.2]octane-2-carboxylate de formule 1, et le schéma réactionnel est présenté ci-dessous, 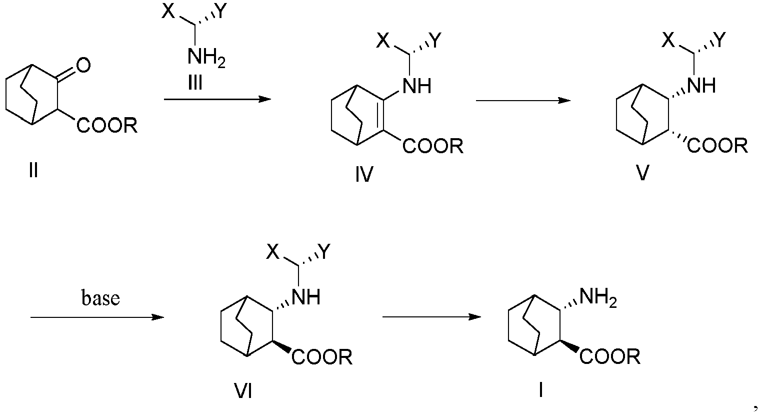 dans lequel,
le R est le méthyle, éthyle, propyle, butyle, phényle ou le benzyle, le X est le méthyle, éthyle, phényle ou le 1-naphtyle, le Y est le méthyle, éthyle, phényle ou le 1-naphtyle, et le X et le Y ne sont pas identiques, le groupe X est plus grand que le groupe Y ;
le procédé comprend :
S1, la mise en réaction du 3-carbonyl-bicyclo[2.2.2]octane-2-carboxylate avec l'amine chirale de formule (III) en présence d'un acide pour donner le 3-amino-bicyclo[2.2.2]octène-2-carboxylate, l'acide est un acide organique fort ;
S2, la réalisation de la réduction avec un agent réducteur ou l'hydrogénation catalysée par un métal du 3-amino-bicyclo[2.2.2]octène-2-carboxylate pour donner le (2R,3S)-3-amino-bicyclo[2.2.2]octane-2-carboxylate ;
S3, dans des conditions fortement basiques, la réalisation de l'inversion de configuration d'ester du (2R,3S)-3-amino-bicyclo[2.2.2]octane-2-carboxylate pour donner le (2S,3S)-3-amino-bicyclo[2.2.2]octane-2-carboxylate, la base forte comprend au moins un choisi parmi le groupe constitué de tert-butylate de sodium, tert-amylate de sodium, diisopropylamide de lithium, bis(triméthylsilyl)amide de lithium, bis(triméthylsilyl)amide de sodium et bis(triméthylsilyl)amide de potassium ; et
S4, la réalisation de l'hydrogénation du (2S,3S)-3-amino-bicyclo[2.2.2]octane-2-carboxylate de formule VI pour éliminer le groupe protecteur pour donner le (2S,3S)-3-amino-bicyclo[2.2.2]octane-2-carboxylate de formule 1.

2. Procédé selon la revendication 1, dans lequel, le R est l'éthyle.

3. Procédé selon la revendication 1, dans lequel, à l'étape S2, l'hydrogénation catalysée par un métal est réalisée en utilisant un catalyseur métallique, le catalyseur métallique comprenant au moins un choisi parmi le groupe constitué de carbone platine, dioxyde de platine et de catalyseur métallique au ruthénium ; ou la réduction est réalisée en utilisant l'agent réducteur, l'agent réducteur comprenant au moins un choisi parmi le groupe constitué de borohydrure de sodium, triacétoxy borohydrure de sodium, cyanoborohydrure de sodium et trifluoroacétoxy borohydrure de sodium.

4. Procédé selon la revendication 1, dans lequel, à l'étape S3, la base forte est le tert-butylate de sodium.

5. Procédé selon la revendication 1, dans lequel, à l'étape S1, l'acide comprend l'acide p-toluènesulfonique ou l'acide trifluoroacétique.

6. Composé de formule V, dans lequel, le R est le méthyle, éthyle, propyle, butyle, phényle ou le benzyle ; le X est le méthyle, éthyle, phényle ou le 1-naphtyle, et le Y est le méthyle, éthyle, phényle ou le 1-naphtyle, et le X et le Y ne sont pas identiques et le X est plus grand que le Y,

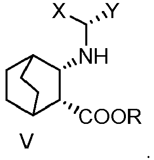

7. Procédé de préparation du (2S,3S)-3-amino-bicyclo[2.2.2]octane-2-carboxylate, dans lequel, le composé V selon la revendication 6 est utilisé dans le procédé.

8. Composé de formule VI, dans lequel, le R est le méthyle, éthyle, propyle, butyle, phényle ou le benzyle ; le X est le méthyle, éthyle, phényle ou le 1-naphtyle, et le Y est le méthyle, éthyle, phényle ou le 1-naphtyle, et le X et le Y ne sont pas identiques et le X est plus grand que le Y,

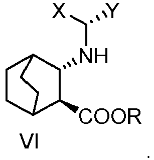

9. Procédé de préparation du (2S,3S)-3-amino-bicyclo[2.2.2]octane-2-carboxylate, dans lequel, le composé VI selon la revendication 8 est utilisé dans le procédé.

10. Procédé selon la revendication 1, dans lequel, le X est un phényle, le Y est un méthyle.

11. Procédé selon la revendication 3, dans lequel, le catalyseur métallique est le dioxyde de platine ; l'agent réducteur est le triacétoxyborohydrure de sodium.

12. Composé de la revendication 6, dans lequel, le R est l'éthyle ; le X est le phényle, le Y est le méthyle.

13. Composé de la revendication 8, dans lequel, le R est l'éthyle ; le X est le phényle, le Y est le méthyle.
